# EUROPEAN PATENT SPECIFICATION

(11) **EP 2 925 777 B1**
(45) Date of publication and mention of the grant of the patent: **01.05.2019**
(21) Application number: 13857951.1
(22) Date of filing: 27.11.2013
(51) Int. Cl.: C07K 14/745

(54) **MONOLITH-BASED PSEUDO-BIOAFFINITY PURIFICATION METHODS FOR FACTOR VIII AND APPLICATIONS THEREOF**
AUF MONOLITH BASIERENDE PSEUDO-BIOAFFINITÄTSREINIGUNGSVERFAHREN FÜR FAKTOR VIII UND ANWENDUNGEN DAVON
PROCÉDÉS DE PURIFICATION PAR PSEUDO-BIOAFFINITÉ À BASE DE MONOLITHE POUR LE FACTEUR VIII ET LEURS APPLICATIONS

(30) Priority: 30.11.2012 IN 5018CH2012
(43) Date of publication of application: 07.10.2015
(73) Proprietor: Centre for Bioseparation Technology-VIT, Tamil Nadu 632014 (IN)
(72) Inventor: JANAKIRAMAN, Vignesh, Narasimhan, Tamil Nadu 632 014 (IN); PRASANNA, Rajasekar, Rajagopal, Tamil Nadu 632 014 (IN); KAMALANATHAN, Agamudi, Sivasankaran, Tamil Nadu 632 014 (IN); VIJAYALAKSHMI, Mookambeswaran, Arunachalam, Tamil Nadu 632 014 (IN)
(74) Representative: V.O.
(86) International application number: PCT/IB2013/060438
(87) International publication number: WO 2014/083510

(56) References cited:
- WO-A1-00/55185
- WO-A1-03/044043
- WO-A2-2014/041483
- WO-A2-2014/041500
- US-B1- 6 831 159
- JEANNE M. LUSHER ET AL: "Evolution of recombinant factor VIII safety: KOGENATE and Kogenate FS/Bayer", INTERNATIONAL JOURNAL OF HEMATOLOGY., vol. 90, no. 4, 1 November 2009 (2009-11-01), pages 446-454, XP055262618, NL ISSN: 0925-5710, DOI: 10.1007/s12185-009-0435-x
- MANTOVAARA T ET AL: "PURIFICATION OF FACTOR VIII C COAGULANT ACTIVITY FROM RAT LIVER NONPARENCHYMAL CELL CULTURE MEDIUM BY IMMOBILIZED METAL ION AFFINITY CHROMATOGRAPHY", BIOTECHNOLOGY AND APPLIED BIOCHEMISTRY, vol. 13, no. 1, 1991, pages 120-126, XP009189336, ISSN: 0885-4513
- SUDHEER REDDY A.R. ET AL: "Expression, Purification, and Partial In Vitro Characterization of Biologically Active Human Coagulation Factor VIII Light Chain (A3-C1-C2) in Pichia pastoris", APPLIED BIOCHEMISTRY AND BIOTECHNOLOGY, vol. 171, no. 1, 30 June 2013 (2013-06-30) , pages 10-19, XP055247043, United States ISSN: 0273-2289, DOI: 10.1007/s12010-013-0338-4
- AMATSCHEK ET AL.: 'Affinity Chromatography of Human Blood Coagulation Factor V lll on Monoliths'with Peptides from a Combinatorial Library' JOURNAL OF HIGH RESOLUTION CHROMATOGRAPHY. vol. 23, no. ISSUE, January 2000, pages 47 - 58, XP002513131
- KELLEY ET AL.: 'Development and Validation of an Affinity Chromatography Step Using a Peptide Ligand for cGMP Production of Factor VIII.' BIOTECHNOLOGY AND BIOENGINEERING. vol. 87, 05 August 2004, pages 400 - 412, XP002424716
- JOSIC D ET AL: "Monoliths as stationary phases for separation of proteins and polynucleotides and enzymatic conversion", JOURNAL OF CHROMATOGRAPHY B: BIOMEDICAL SCIENCES & APPLICATIONS, ELSEVIER, AMSTERDAM, NL, vol. 752, no. 2, 10 March 2001 (2001-03-10), pages 191-205, XP004317155, ISSN: 1570-0232, DOI: 10.1016/S0378-4347(00)00499-0
- VIJAYALAKSHMI MOOKAMBESWARAN A: "Histidine ligand affinity chromatography", MOLECULAR BIOTECHNOLOGY, HUMANA PRESS, INC, US, vol. 6, no. 3, 1 January 1996 (1996-01-01) , pages 347-357, XP009501297, ISSN: 1073-6085, DOI: 10.1007/BF02761713

## Description

### TECHNICAL FIELD

The present invention relates to purification of Factor VIII protein and/or its fragments from various sources by employing monolith based pseudo-bioaffinity purification methods. In particular, L-histidine over CIM monolith [i.e. Histidine Ligand Affinity Chromatography (HLAC)] and immobilized metal over CIM monolith [Immobilized metal-ion affinity chromatography (IMAC)] for the purification of factor VIII and/or its fragments from various sources is described.

### BACKGROUND OF THE INVENTION

Factor VIII:C (FVIII:C) is an essential blood coagulation factor which plays a key role in the pathology of haemophilia. FVIII:C is a plasma protein essential for blood coagulation whose deficiency or defective formation results in the blood clotting disorder known as Haemophilia A. FVIII:C is synthesized as a 300-kd precursor protein comprising of six domains: A1, A2, B, A3, C1 and C2. In the plasma, it circulates as a heterodimer consisting of the heavy chain (A1-A2-B domains) and light chain (A3-C1-C2 domains) as a result of limited proteolysis by thrombin. FVIII:C is activated by further proteolysis of the heavy chain resulting in a heterotrimer consisting of A1, A2 and A3-C1-C2 subunits, which in turn activates downstream zymogens completing the coagulation cascade resulting in a clot.

To counter the loss of factor VIII:C activity observed in haemophilia patients, factor VIII:C is administered as plasma concentrates or as recombinant factor VIII:C expressed using mammalian cells. However, the key difficulty associated with both these methods is the efficient purification and recovery of factor VIII:C from their respective sources.

Classical affinity-based purification methods using antibodies specific to the factor VIII:C molecule are conventionally employed for FVIII:C purification. However, said methods often results in loss of activity due to the harsh elution conditions employed which leads to structural variations in factor VIII:C. It is well known in the art that in order to carry out functional studies and for practical applications, the expressed factor VIII:C protein needs to be purified without significant loss of its activity. But, the currently employed methods for FVIII:C purification have several drawbacks which lead to the loss of FVIII:C activity.

The replacement therapy for haemophilia patients using plasma concentrates of factor VIII:C or recombinant factor VIII:C which are currently employed to counter/treat haemophilia A requires improvement in terms of better purity and efficient recovery of factor VIII:C. Such efficient purification and recovery of factor VIII:C translates to money and lives saved. However, as described above, the conventional purification of factor VIII:C involve affinity-based methods which employ harsh elution conditions which may alter the structure of the protein thereby reducing biological activity.

Thus, it can be observed that the presently employed FVIII purification methods are associated with various drawbacks and hence, there is an immense need to arrive at better purification methods for purifying FVIII protein from various sources.

The present invention aims at overcoming all the aforesaid drawbacks of the prior art and providing efficient purification methods for Factor VIII.

### STATEMENT OF INVENTION

Accordingly, the present invention relates to a method of purifying Factor VIII protein or fragment thereof from a sample, said method consisting of subjecting the sample to monolith based pseudo-bioaffinity purification to obtain said purified Factor VIII protein or fragment thereof, wherein the monolith based pseudo-bioaffinity purification comprises act ofa) coupling or immobilizing a monolith column with a ligand selected from a group consisting of L-histidine and chelated transition divalent metal ion selected from a group comprising copper, nickel, cobalt, zinc and iron to obtain an immobilized monolith column; b) equilibrating the immobilized monolith column obtained in step (a) and loading the sample into the column; c) optionally carrying out a wash step; and d) eluting the sample to obtain the Factor VIII protein or fragment thereof.

### BRIEF DESCRIPTION OF ACCOMPANYING FIGURES

In order that the invention may be readily understood and put into practical effect, reference will now be made to exemplary embodiments as illustrated with reference to the accompanying figures. The figures together with a detailed description below, are incorporated in and form part of the specification, and serve to further illustrate the various embodiments, principles and advantages, in accordance with the present invention where:

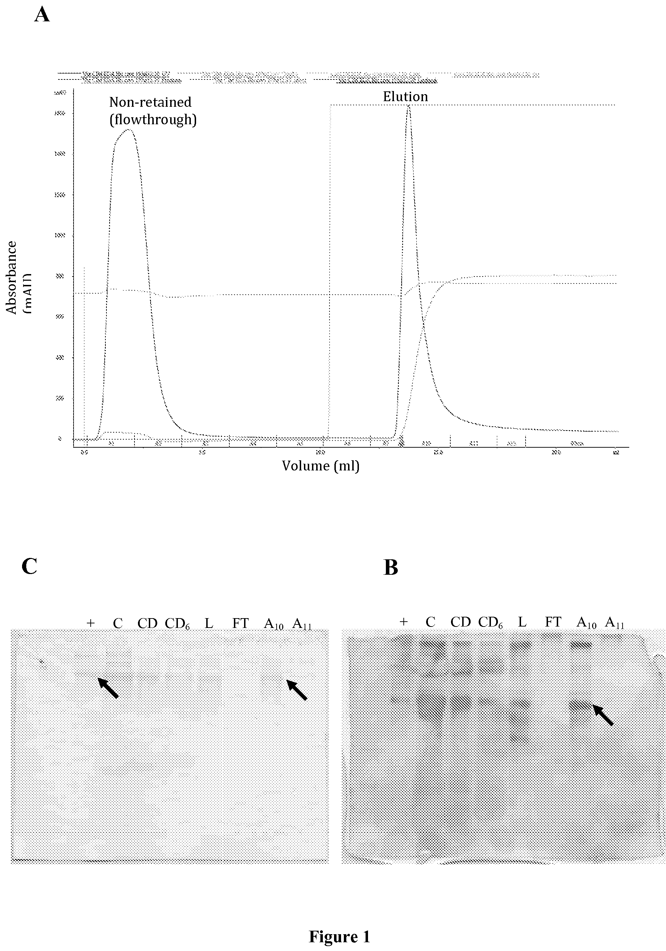

**Figure 1** depicts the chromatography results of plasma cryoprecipitate over CIM-EDA-L-Histidine (i.e. HLAC purification method). Chromatogram (**A**), 8% SDS-PAGE analysis under non-reducing conditions (**B**) and Western blotting results (**C**) are depicted. Different lanes of the SDS-PAGE gel (**B**) and Western blot (**C**) correspond to- plasma cryoprecipitate (C), dialysed cryoprecipitate at pHs 7.0 and 6.0 (CD, CD₆), load (L), unbound fraction (Flow-through, FT) and eluted fractions (A₁₀, A₁₁). Pure factor VIII:C is used as control (+). The Mol. Wt. marker used in the gel is a medium-range protein marker. The Mol. Wt. of full length FVIII is about 220 kDa. Arrows on the nitrocellulose membrane (figure 1**C**- Western Blot) indicate the presence of FVIII protein.

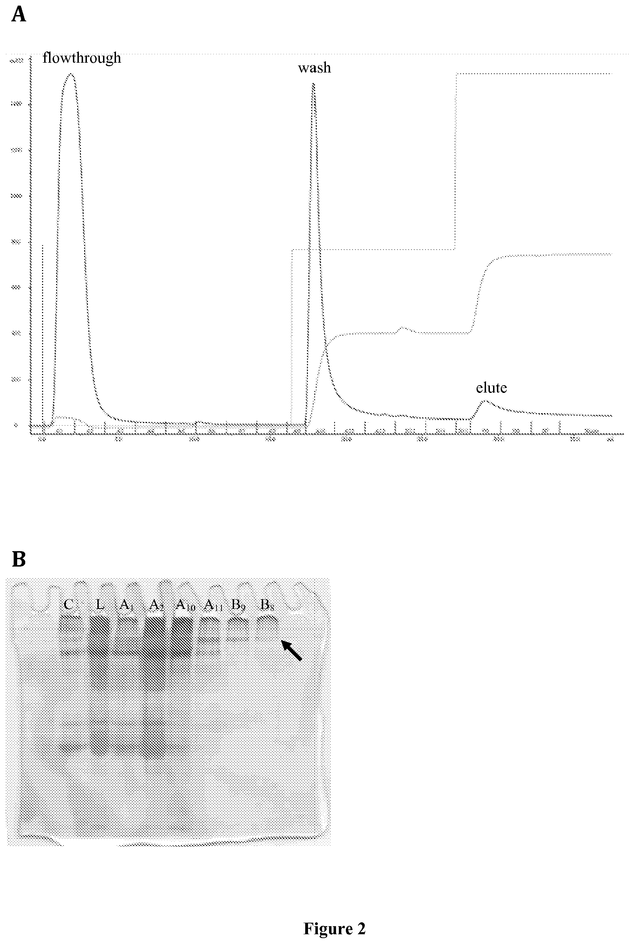

**Figure 2** depicts the chromatography of plasma cryoprecipitate over CIM-EDA-Histidine (HLAC) after performing an intermediary wash step. Chromatogram (**A**) and 8% SDS-PAGE analysis under non-reducing conditions (**B**) is depicted. Different lanes of the SDS-PAGE gel (**B**) correspond to plasma cryoprecipitate (**C**), load (L), unbound fractions (A₁, A₂), elution at 50% elution buffer (A₁₀, A₁₁) and 100% elution buffer (B₉, B₈).

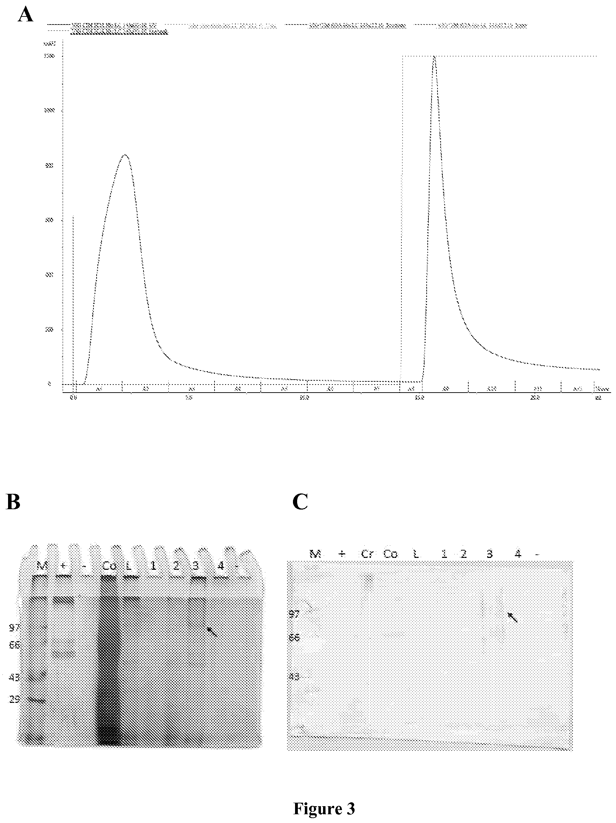

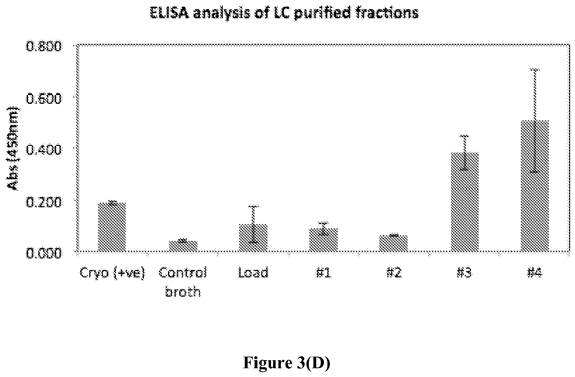

**Figure 3** depicts chromatography of Factor VIII:C light chain over CIM-EDA-Histidine (HLAC). Chromatogram (**A**) and 10% SDS-PAGE (**B**) & western blot (**C**) analysis under non-reducing conditions are depicted. Different lanes of the SDS-PAGE gel (**B**) and western blot (**C**) highlight the Load (L), Unbound fractions (1, 2) and Eluted fractions (3, 4), in addition to commercial purified Factor VIII:C (+, used as positive control) and a control Pichia broth (Co) not expressing the light chain protein. Further, figure 3(**D**) depicts the ELISA results performed on various fractions.

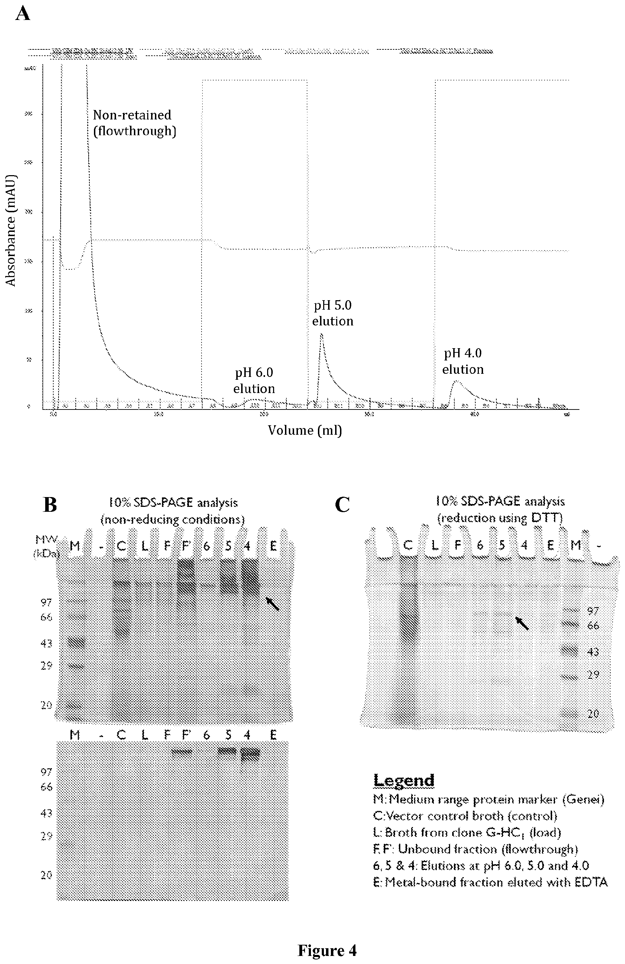

**Figure 4** depicts the purification results of heavy chain FVIII:C over CIM-IDA-Cu (i.e. IMAC). The figure showcases chromatogram (**A**), SDS-PAGE and Western Blotting analysis under non-reducing conditions (**B**) and SDS-PAGE under reducing conditions (**C**). Further, the lanes of 4 (**B**) correspond to control Pichia broth which doesn't express heavy chain (-), load (L), unbound fractions (flowthrough) (F, F'), bound fractions eluted at pHs 6.0/5.0/4.0 (6,5,4) and EDTA-eluted fraction (E). The FVIII:C heavy chain is obtained to near homogenity upon elution at pH 5.0.

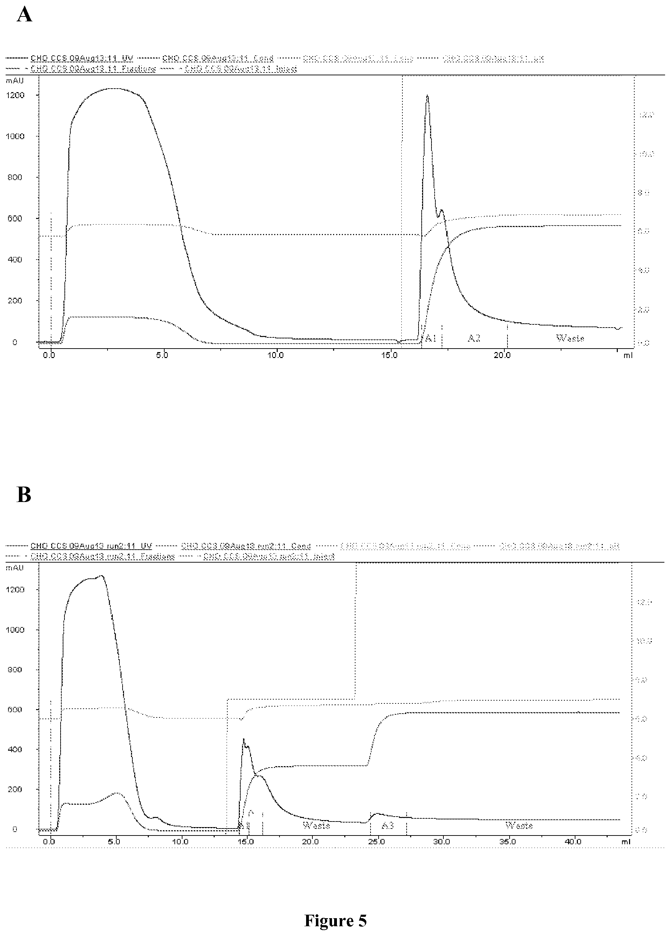

**Figure 5** depicts the HLAC purification results of recombinant BDD-FVIII expressed in CHO cells. The figure showcases chromatographic profile without wash step (**A**) and with wash step (**B**).

### DETAILED DESCRIPTION OF THE INVENTION

The present invention relates to a method of purifying Factor VIII protein or fragment thereof from a sample, said method comprising act of subjecting the sample to monolith based pseudo-bioaffinity purification to obtain said purified Factor VIII protein or fragment thereof, wherein monolith based pseudo-bioaffinity purification comprises act of
a) coupling or immobilizing a monolith column with a ligand selected from a group consisting of L-histidine and chelated transition divalent metal ion selected from a group comprising copper, nickel, cobalt, zinc and iron to obtain an immobilized monolith column;
b) equilibrating the immobilized monolith column obtained in step (a) and loading the sample into the column;
c) optionally carrying out a wash step; and
d) eluting the sample to obtain the Factor VIII protein or fragment thereof.

In an embodiment of the present invention, the Factor VIII protein or fragment thereof within the sample is naturally occurring or recombinant Factor VIII protein or fragment thereof, or a combination thereof.

In another embodiment of the present invention, the natural Factor VIII protein or fragment thereof is obtained from a source selected from a group comprising plasma and liver or a combination thereof, and the recombinant Factor VIII protein or fragment thereof is obtained from a cell selected from a group comprising CHO cell, *Pichia pastoris, Lemna gibba* and *Hansunella polymorpha,* or any combination thereof.

In yet another embodiment of the present invention, the Factor VIII fragment is Factor VIII Light Chain, Factor VIII heavy chain, or a combination thereof.

In still another embodiment of the present invention, the Factor VIII heavy chain has a molecular weight ranging from about 90 kDa to 210 kDa; and wherein light chain has a molecular weight of about 80 kDa.

In still another embodiment of the present invention, the method purifies Factor VIII protein or fragment thereof with purification factor value ranging from about 295 fold to about 6179 fold.

In still another embodiment of the present invention, the yield of purified Factor VIII protein or fragment thereof ranges from about 15 % to about 55 %.

In still another embodiment of the present invention, the ligand is selected from L-histidine or transition metal ion, or a combination thereof.

In still another embodiment of the present invention, the monolith based pseudo-bioaffinity purification is Histidine Ligand Affinity Chromatography (HLAC) when the ligand is L-histidine, and wherein the monolith based pseudo-bioaffinity purification is Immobilized metal-ion affinity chromatography (IMAC) when the ligand is transition metal ion.

In still another embodiment of the present invention, the transition metal ion is selected from a group comprising copper, nickel, cobalt and zinc, or any combination thereof.

In still another embodiment of the present invention, the monolith column is Convective Interaction Media (CIM) monolithic column.

In still another embodiment of the present invention, the coupling or immobilization is carried out in presence of coupling agent selected from a group comprising ethylene-di-amine (EDA), carbonyldiimidazole (CDI), epoxy, imino-di-acetic acid (IDA) and Tris(2-aminoethyl)amine (TREN), or any combination thereof.

In still another embodiment of the present invention, the equilibration is carried out by employing buffer selected from a group comprising cationic buffer, phosphate buffer, 3-(N-morpholino)propanesulfonic acid (MOPS) buffer and MMA buffer, or any combination thereof, and wherein the phosphate buffer, MOPS buffer and MMA buffer contain salt at concentration ranging from about 0.5 M to about 2 M, preferably sodium chloride at concentration of about 0.5 M.

In still another embodiment of the present invention, concentration of the aforesaid cationic buffer ranges from about 20 mM to about 100 mM and pH ranges from about 5.5 to about 6.5, and wherein concentration of the aforesaid phosphate buffer, MOPS buffer and MMA buffer ranges from about 20 mM to about 100 mM and pH ranges from about 7.0 to about 8.0.

In still another embodiment of the present invention, the elution is carried out by employing buffer selected from a group comprising cationic buffer containing calcium (II) ions, glycinate ions and lysine, acetate buffer containing sodium chloride, or a combination thereof.

In still another embodiment of the present invention, concentration of the cationic buffer as mentioned for elution ranges from about 20 mM to about 100 mM and pH ranges from about 7 to about 8, and wherein concentration of the acetate buffer ranges from about 20 mM to about 100 mM and pH ranges from about 4 to about 5.

In still another embodiment of the present invention, the wash step is carried out by employing buffer selected from a group comprising cationic buffer, phosphate buffer, 3-(N-morpholino)propanesulfonic acid (MOPS) buffer and MMA buffer, or a combination thereof. In still another embodiment of the present invention, concentration of the cationic buffer as mentioned for wash step ranges from about 10 mM to about 50 mM and pH ranges from about 7 to about 8, and wherein concentration of the phosphate buffer, 3-(N-morpholino)propanesulfonic acid (MOPS) buffer and MMA buffer ranges from about 20 mM to about 100 mM and pH ranges from about 6.0 to about 7.0.

In still another embodiment of the present invention, the cationic buffer as mentioned above for equilibration, elution and wash steps is selected from a group comprising Tris-HCl, and bicarbonate, or a combination thereof.

The present disclosure further relates to a purified, biologically active Factor VIII protein or fragment thereof obtained by the above method, wherein the method purifies Factor VIII protein or fragment thereof with purification factor value ranging from about 30 fold to about 6179 fold.

Pseudobioaffinity-based purification methods are used to obtain proteins of our interest by employing mild elution conditions, thus avoiding protein denaturation. Said pseudo-bioaffinity based purification methods involve interaction between proteins and immobilized metals or proteins and histidine which are made specific under optimal conditions to enable single-step purification of proteins from biological mixtures.

In the present invention two pseudobioaffinity-based purification methods namely, Histidine Ligand Affinity Chromatography (HLAC) and Immobilized Metal Affinity Chromatography (IMAC) are employed for purification of FVIII protein. HLAC is used for the purification of factor VIII:C from various sources such as plasma cryoprecipitate, recombinant B-domain deleted factor VIII:C expressed in CHO cell lines and recombinant factor VIII:C light chain expressed in *Pichia pastoris.* Similarly, IMAC is employed for the purification of recombinant factor VIII:C from several sources such as plasma cryoprecipitate, recombinant B-domain deleted factor VIII:C expressed in CHO cell lines and recombinant factor VIII:C heavy chain expressed in *Pichia pastoris.* In an embodiment, the molecular weight of full-length FVIII ranges from about 170kDa to about 290kDa, depending on the extent of proteolysis of the heavy chain. Further, the molecular weight of BDD-FVIII used in the present invention has a molecular weight of about 170kDa.

Histidine ligand affinity chromatography (HLAC), is employed by the present invention to retain Factor VIII and/or its fragments (preferably, Factor VIII-Light Chain) with high selectivity, based on a combined hydrophobic and ion pairing complementarity. This high selectivity is possible due to the uniqueness of the amino acid L-Histidine: its mild hydrophobicity, weak charge transfer possibilities and wide pKa range. These factors result in a multimodal interaction of weak to average binding strength, which would in-turn facilitate mild adsorption and elution conditions ensuring structural integrity of the Factor VIII and/or its fragments.

The present invention successfully employs pseudo-bioaffinity based purification methods involving the amino acid L-histidine as ligand (i.e. HLAC) over CIM monolith and immobilized metal ions (i.e. IMAC) over CIM monolith to efficiently recover factor VIII:C and/or its fragments from various sources using minimal steps, preferably in one step. L-Histidine as ligand coupled to a monolith-based Convective Interaction Media (CIM) support system (CIM-HLAC) is employed to purify factor VIII:C from plasma and various other sources as described above. The CIM stationary phase pose several advantages over other conventional support systems, as they exhibit high dynamic binding capacity for large molecules, flow independent performance and low pressure drop even at high flow rates which enable rapid separation. CIM monoliths are made of monolithic polymethacrylate polymers. These CIM monoliths, or Short Monolithic Columns (SMCs) are prepared by free radical polymerization of a mixture of glycidyl methacrylate (providing functional groups), ethylene dimethacrylate (as a cross-linking reagent), 2,2'-azobisisobutyronitrile (as an initiator) and a porogenic solvent (cyclohexanol and dodecanol) in barrels of polypropylene syringes, yielding glycidyl methacrylate-co-ethylene dimethacrylate (GMA-EDMA) monoliths. After polymerization, the block of polymer formed in disk or tube shape, is mounted in a specially designed housing allowing good sample distribution and low dead volume.

Table 1 highlights some of the advantages of CIM monolithic supports over conventionally packed columns.

**Table 1: Comparison of monolithic supports with conventionally packed columns**

| **Chromatographic Support** | **Nature of flow** | **Binding efficiency** | **Scale up** | **Range of accepted proteins** |
|---|---|---|---|---|
| Conventionally packed columns | Diffusion-based | Restricted to low flow rates due to back pressure | Difficult | Restricted by pore size |
| Monoliths | Convective | Independent of flow rates | Easy | Accommodates large proteins |

Thus, by combining the ultra-fast flow system of CIM with pseudobioaffinity based ligand L-Histidine or immobilized metal ions, the present invention provides for quick and efficient purification of factor VIII and/or its fragments from various sources.

In an embodiment of the present invention, Histidine Ligand Affinity Chromatography (HLAC) over CIM support is used for the rapid purification of FVIII:C from plasma.

In another embodiment of the present invention, Histidine Ligand Affinity Chromatography (HLAC) over CIM support is used for the rapid purification of recombinantly expressed FVIII light chain (FVIII-LC).

In yet another embodiment of the present invention, HLAC over CIM support is used for the rapid purification of recombinantly expressed B-domain deleted (BDD)/full-length FVIII from various host systems not limited to CHO cell lines, *Pichia pastoris, Lemna gibba* and *Hansenula polymorpha.*

In an exemplary embodiment of the instant invention, the purification of FVIII-LC or BDD/full length FVIII by HLAC occurs by the binding of the ligand L-Histidine to the light chain of FVIII or BDD/full length FVIII wherein, the binding mechanism of BDD/full length FVIII protein to L-Histidine is through the light chain. L-Histidine interacts with BDD/full length FVIII in a multi-modal manner, owing to its unique properties: wide pKa range, mild hydrophobicity and weak charge transfer possibilities. Binding of FVIII to L-Histidine is favoured under mild acidic conditions, and elution is achieved upon raising the pH to near-neutral levels along with the addition of calcium (II) ions, which stabilizes the FVIII molecule. While the light chain of factor VIII is expected to bind to L-Histidine by both positive and negative ionic forces, the retention of L-Histidine is primarily through positive forces of binding based on surface exposed charges. The addition of lysine in the elution buffer helps break these specific ionic bonds.

In yet another embodiment of the present invention, Immobilized Metal-ion Affinity Chromatography (IMAC) over CIM monoliths is employed to rapidly purify factor VIII protein and/or its fragments from various natural and recombinant sources. In an exemplary embodiment, IMAC is employed to purify recombinantly expressed heavy chain of factor VIII:C. The rationale behind employing IMAC in the present invention is based upon the examination of the 3-dimentional structure of the heavy chain of factor VIII:C which reveals 3-4 Histidine residues exposed on the surface of the heavy chain. The principle of metal chelating with the exposed surface having histidine is employed here, without the need for any additional affinity tags. In other words, the immobilised metal affinity chromatography (IMAC) technique takes advantage of the property of certain amino acids like Histidine to bind to chelated transition divalent metal ions (such as copper, nickel, cobalt, zinc or iron). This binding is facilitated at near neutral pH, wherein the deprotonated imidazole ring of the exposed Histidine residues form coordinate bond with the immobilized metal. Lowering the pH of the buffer results in protonation of imidazole, leading to a break in the coordinate bond subsequently elution of the protein.

Therefore, the present invention successfully employs pseudo-bioaffinity based purification methods involving the amino acid L-histidine as ligand (i.e. HLAC) over CIM monolith and immobilized metal ions (i.e. IMAC) over CIM monolith to rapidly and efficiently purify factor VIII:C and/or its fragments from various sources using minimal steps.

Additional embodiments and features of the present invention will be apparent to one of ordinary skill in art based upon description provided herein. However, the examples and the figures should not be construed to limit the scope of the present invention.

### Materials Used for arriving at the Examples of the present invention

Chromatographic procedures involving plasma-factor VIII:C and recombinant factor VIII:C light chain are carried out using CIM (Convective Interaction Media) monolithic disk (BIA separations d.o.o., Slovenia), coupled with L-Histidine as ligand using a new coupling chemistry. Chromatographic procedures involving recombinant factor VIII:C heavy chain are carried out over CIM-IDA monolithic disk chelated with Cu⁺⁺ ions (ligand). Protein samples obtained from chromatographic procedures are concentrated using Amicon® Ultra-4 Centrifugal Filter Units (Millipore, Billerica, USA). Buffers for chromatographic runs and reagents are prepared using chemicals of analytical grade either from Sigma-Aldrich (St. Louis, USA) or HiMedia (Bangalore, India).

Protein purification (chromatographic procedures) is performed using ÄKTA FPLC workstation from GE Healthcare and is monitored with Unicorn 5.1 software (GE Healthcare, Uppsala, Sweden). Protein detection is monitored at 280nm. Further, spectrophotometric measurements of samples are made using Beckman Coulter DU730 UV-Vis Spectrophotometer (Beckman Coulter, Hope City, USA).

Plasma cryoprecipitate for purification of factor VIII:C is obtained from Department of Haematology, Christian Medical College (CMC), Vellore. The recombinant factor VIII:C heavy chain and light chains are expressed in house using the yeast expression host *Pichia pastoris* GS115.

### Example 1

### Purification of Factor VIII:C from plasma

Plasma can be employed as a direct natural source for obtaining FVIII protein. Alternatively, plasma cryoprecipitate can be prepared and used as a source of FVIII.

In a specific embodiment, the plasma cryoprecipitate [which is the precipitate obtained by the freezing of fresh-frozen plasma followed by its thawing at 4°C] is pre-treated by dialysing the said cryoprecipitate against 20mM Tris, pH 7.0 over a period of about 6 hours at about 4°C. The pH of the dialysed sample is reduced using 20mM Tris at pH 6.0 which results in the formation of a precipitate. The sample is centrifuged at 10,000g for about 5 minutes, and the supernatant is used as source material for the chromatographic run.

Purification of factor VIII:C from dialysed plasma cryoprecipitate is carried out by HLAC using the amino acid L-Histidine as ligand. L-Histidine can be immobilized/coupled to CIM support through a number of coupling groups, including epoxy, ethylene-di-amine (EDA) and carbonyldiimidazole (CDI) chemistries. The inventors of the present invention have successfully coupled L-Histidine through all three chemistries, wherein the coupling/immobilization using EDA chemistry is described in detail. A fresh CIM-EDA disk is equilibrated with 50mM Phosphate buffer, pH 7.5 at a flow rate of about 1ml/min for about 1 hour at room temperature. About 30ml of 10% glutaraldehyde solution (v/v) in 50mM phosphate buffer, pH 7.5 is pumped through the disk, after which the disk is incubated with about 5ml of the same glutaraldehyde solution overnight in dark at about 25° C for derivatization in a glass beaker. Following derivatization, the disk is washed with about 30 ml of 0.5M phosphate buffer pH 7.5, followed by a second wash with 0.5M phosphate buffer, pH 3.0 for about 1 hour at a flow rate of about 1ml/min. About 20 ml of L-Histidine solution (about 50mg/ml L-Histidine monohydrochloride monohydrate in 0.5M phosphate buffer, pH 3.0) is then pumped through the column at a flow rate of about 0.4 ml/min for about 6 hours under recirculation, after which the disk is incubated in the L-Histidine solution in a glass beaker for about 24 hours at 25 °C under constant gentle shaking (about 75-80 rpm). The disk is then washed with about 10 ml of 0.5M phosphate buffer, pH 3.0 followed by about 10 ml of 0.1M cyanoborohydride solution which is prepared by dissolving in 0.5M phosphate buffer, pH 3.0. The disk is thereafter incubated with about 10 ml of the same cyanoborohydride solution for about 3 hours at 25°C under gentle shaking, after which the excess cyanoborohydride is washed off with 0.5M phosphate buffer pH 3.0. Endcapping or blocking of residual reactive groups in the support matrix, is achieved by passing about 10ml of 1M monoethanolamine in about 50mM phosphate buffer, pH 7.5 followed by incubation of the disk in about 5 ml of monoethanolamine solution for about 3 hours at 25°C. The disk is finally washed with 50mM phosphate buffer, pH 7.8 followed by a wash with 1M NaCl in 25mM Tris-HCl, pH 7.4. This CIM disk immobilized with L-Histidine (CIM-EDA-L-Histidine) is used for further purification experiments.

The prepared CIM- EDA-L-Histidine disk (dimensions: 12mm×3mm, volume: 0.34 ml) is placed into the housing to obtain a CIM monolithic column. The binding and elution flow rates are varied between a range of about 3 CV/min to about 18 CV/min. The column is equilibrated using 20mM-100mM of cationic buffers such as Tris-HCl at pH 5.5 to 6.5, preferably 20mM Tris buffer at pH 6.0 ± 0.2, and the sample is injected. Elution is performed using 20mM-100mM of cationic buffers such as Tris-HCl at pH 7.0 to 8.0, in the presence of Calcium (II) ions, Glycinate ions and Lysine. Preferably, elution is performed using an optimized step gradient of 20mM Tris buffer, pH 7.0 containing 0.1M Glycine, 0.03M Lysine and 0.3M CaCl₂. The purified fractions are further confirmed by SDS-PAGE and western blotting analysis for the protein of interest and are assayed for their biological activity by performing clotting assays.

As described above, SDS-PAGE, followed by western blotting is performed on purified samples to confirm the presence of factor VIII:C protein. About 5 µg of protein sample is loaded on each well of a polyacrylamide gel (8% polyacrylamide gels), and SDS-PAGE is performed to separate them. After electrophoresis, the proteins from the polyacrylamide gel are transferred to a nitrocellulose membrane using Mini Trans-Blot Cell (BIO-RAD, India). Towbin transfer buffer (25mM Tris, 200mM glycine, 0.01% SDS, 20% methanol, pH 8.3) is used to carry out the transfer from polyacrylamide gel to the nitrocellulose membrane, at 90V for about 90 minutes at a temperature of about 4°C. The membrane is thereafter washed with PBST (Phosphate Buffered Saline with 0.1% Tween-20), blocked with a blocking buffer (PBST with 5% skimmed milk powder) overnight at a temperature of about 4°C. After blocking, the membrane is incubated with primary antibody (rabbit anti-A₁ or anti-C₂ antiserum) at room temperature for about one hour. The membrane is thereafter washed for about 2-4 times with PBST solution after which it is incubated with secondary antibody (goat anti-rabbit IgG, HRP conjugated) at room temperature for about 1 hour. The membrane is then washed for about 2-4 times with PBST. The membrane is finally developed by diaminobenzidine/H₂O₂ method for detection of HRP.

The rabbit anti-A₁ and anti-C₂ antisera used as above for the detection of the expressed proteins by western blotting are prepared using *E. coli* expressed heavy and light chain terminal domains A₁ and C₂ domains respectively, tagged with GST as antigen. Rabbits are immunized with the purified antigens (A₁-GST and C₂-GST). The antiserum generated to one of these two antigens is used to probe the western blots.

### Purification Results

Plasma cryoprecipitate is obtained, pretreated and the sample is injected over an equilibrated CIM-EDA-L-Histidine disk. The chromatogram obtained after HLAC purification is depicted in Figure 1A. The chromatogram clearly shows the signal corresponding to non-retained proteins flowing through upon sample injections followed by the recovery of factor VIII:C in a sharp peak obtained by passing the elution buffer through the disk. Further, upon SDS-PAGE analysis of the purified fraction (Fig. 1B), a clear band is observed in the tail portion of the eluted fraction (A₁₀), which exhibits substantial coagulation activity verified by clotting assays (Table 2). The purification of Factor VIII:C protein is further confirmed by Western Blot results wherein the A₁₀ fraction clearly shows the presence of Factor VIII:C (Fig. 1C).

### Activity Results

The coagulation activity of different fractions during the purification process is measured using one stage clotting assay. The one stage clotting assay is performed as follows-

Factor VIII-depleted plasma is dissolved in distilled or deionized water. Before use, it is allowed to stand for at least 15 minutes at a temperature of about 15 to 25°C, and then mixed carefully without foam formation. The sample (one of various fractions, viz., cryoprecipitate, processed cryoprecipitate, load or elute obtained at different stages of the purification process) is added to FVIII deficient plasma. Activated Partial Thromboplastin Time (APTT) reagents are used according to the manufacturer's instructions for determining the coagulation activity. 0.025M of CaCl₂ is added to the mixture. The mixture is incubated at a temperature of about 37°C for about 2 minutes and the reading is taken in an automated coagulation analyzer. Normal clotting time is 30-40 seconds.

The respective specific activity results are showcased in Table 2 below.

**Table 2: Activity table for purification of factor VIII:C from plasma cryoprecipitate**

| **Total Units** | **Specific Activity** | | |
|---|---|---|---|
| 87.7 (0.5mL) | 14.0 IU/mg | | Cryoprecipitate |
| | | 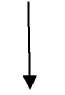 | Dialysis with 20mM Tris, pH 6.0 |
| 8.85 (0.5mL) | 1.5 IU/mg | Processed Cryoprecipitate | |
| | | 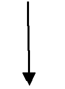 | |
| 0.65 (2.0mL) | 1.4 IU/m | Load | |
| | | 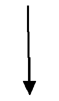 | Chromatography over CIM-EDA-His |
| 12.8 (2.0mL) | 412.5 IU/mg | Elute (A₁₀) | |

In an embodiment, about 295-fold purification of Factor VIII protein is achieved with a recovery/yield of about 15%. A fraction of the protein is lost in the non-retained fraction (i.e. flow through), which is due to the lower capacity of the CIM-0.34mL disk. The said protein can be recovered by rechromatographing the flow-through fraction again, or by using a larger disk/column.

In another embodiment of the present invention, the HLAC purification process is further streamlined by the inclusion of an intermediate wash step at 50% to remove the adjoining impurities (Figure 2). By the introduction of an intermediary wash step using 50% elution buffer, other proteins from the Factor VIII:C mixture gets separated leading to improved purification. In yet another embodiment, the higher purification results achieved due to intermediary wash step is further substantiated by the marked increase in activity exhibited by the eluted fraction B₈ (Figure 2B and Table 3). The results showcase an increased recovery/yield of 55% and about 1386-fold increase in purity. A fraction of Factor VIII:C protein is lost in the non-retained fraction (flow-through). Hence, the percentage of recovery/yield can be further improved by passing the flow through fraction through the column/disk again, or by using a larger disk/column.

**Table 3: Activity table for purification of factor VIII:C from plasma cryoprecipitate (with intermediary wash step)**

| **Total Units** | **Specific Activity** | | |
|---|---|---|---|
| 87.7 (0.5mL) | 14.0 IU/mg | Cryoprecipitate | |
| | | 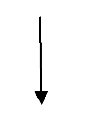 | Dialysis with 20mM Tris, pH 6.0 |
| 10.8 (2.0mL) | 0.5 IU/mg | Load | |
| | | 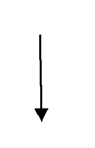 | Chromatography over CIM-EDA-His with intermediary wash step |
| 48.5 (2.0mL) | 693.0 IU/mg | Elute (B₈) | |

Based on the above results, it is apparent that Factor VIII protein has been purified to high purity from plasma cryoprecipitate using L-Histidine immobilized on CIM support.

### Example 2

### Purification of recombinant Factor VIII:C light chain

Purification of factor VIII:C light chain (80 kDa) expressed by *Pichia pastoris* GS115 cells is carried out using CIM-His disk. The CIM-His disk (dimensions: 12mm×3mm, volume: 0.34 ml) is prepared as described in the previous example and the same is placed into the housing to obtain a CIM monolithic column. Constant binding and elution flow rates ranging between 3 CV/min to 18 CV/min are employed throughout the experiment. The column is equilibrated using 20mM-100mM of cationic buffers such as Tris-HCl at pH 5.5 to 6.5, preferably with 20 mM Tris buffer at pH 6.0, and the sample (*Pichia* broth expressing factor VIII:C light chain concentrated by 0→80% saturation ammonium sulphate) is injected. Elution is performed in a single step using 20mM-100mM of cationic buffers such as Tris-HCl at pH 7.0 to 8.0, in the presence of Calcium (II) ions, Glycinate ions and Lysine. Preferably, elution is performed using 20mM Tris buffer, pH 7.0 containing 0.1M Glycine, 0.03M Lysine and 0.3M CaCl₂ to obtain purified fractions.

SDS-PAGE, followed by western blotting is performed on purified samples to confirm the presence of factor VIII:C light chain. About 5µg of obtained protein sample is loaded on each well of a polyacrylamide gel (10% polyacrylamide gels), and SDS-PAGE is performed. After electrophoresis, the proteins from the polyacrylamide gel are transferred to a nitrocellulose membrane using Mini Trans-Blot Cell. Towbin transfer buffer (25mM Tris, 200mM glycine, 0.01% SDS, 20% methanol, pH 8.3) is used to carry out the transfer from the polyacrylamide gel to the nitrocellulose membrane at 90V for about 90 mins at a temperature of about 4°C. The membrane is washed with PBST (Phosphate Buffered Saline with 0.1% Tween-20), blocked with a blocking buffer (PBST with 5% skimmed milk powder) overnight at a temperature of about 4°C. After blocking, the membrane is incubated with primary antibody (rabbit anti-A₁ or anti-C₂ antiserum, along with excess of control *Pichia* broth to prevent non-specific binding) at room temperature for about one hour. The membrane is washed 2-4 times with PBST (Phosphate Buffered Saline solution with the detergent Tween 20) solution after which it is incubated with secondary antibody (goat anti-rabbit IgG, HRP conjugated) at room temperature (25°C- 30°C) for about 1 hour. The membrane is thereafter washed 2-4 times with PBST. Finally, the membrane is developed by diaminobenzidine/H₂O₂ method for detection of HRP.

The rabbit anti-C₂ antiserum is used to follow the purification of the factor VIII:C light chain protein by western blotting is prepared using *E. coli* expressed light chain terminal domain C₂ domain, tagged with GST, as antigen. Rabbits are immunized with the purified antigen (C₂-GST). During addition of rabbit antisera to the membrane, the antiserum is incubated along with excess of *Pichia pastoris* control broth to reduce non-specific binding and background noise.

### Purification Results

Histidine Ligand Affinity Chromatography (HLAC) is employed to purify the recombinantly expressed light chain of FVIII:C in *Pichia pastoris* as described above. The load, flow through and eluted fractions of the chromatographic experiment are analyzed over 10% SDS-PAGE under non- reducing conditions. Upon SDS-PAGE and western blotting analysis under non-reducing conditions, a band corresponding to the light chain protein is observed in the eluted fraction (lane 3, Figures 3B and 3C). The eluted fraction shows a band slightly above the expected 80 kDa. This increase in the theoretically predicted molecular weight could be attributed to glycosylation on the light chain residues. Nonetheless, the purification and presence of FVIII-Light Chain is successfully confirmed by ELISA analysis [Figure 3(D)].

This purified factor VIII:C light chain is used for reconstitution with purified heavy chain to exhibit coagulation activity.

### Example 3

### Purification of recombinant Factor VIII:C heavy chain

Purification of heavy chain of factor VIII:C from the heavy chain expressing *Pichia pastoris* clone is carried out using immobilized metal ion affinity chromatography (IMAC) using Cu⁺⁺ ions as ligand. The Cu⁺⁺ ions are chelated on to the CIM-IDA [CIM-Imino-Di-Acetic acid] disk (dimensions: 12mm×3mm, volume: 0.34 ml), and packed into the housing to obtain a CIM monolithic column. Chelation of Cu++ ions on the CIM-IDA disk is achieved by passing about 20 column volumes of 50mM CuSO₄ over the CIM-IDA disk. Further, the principle of retention of the heavy chain protein by CIM-Cu column is based on the coordinate bond formed between the immobilized divalent transition metal ion (ligand) and the histidine residue of the heavy chain protein. When the pH of the buffer is reduced, the charge on the histidine residue on the heavy chain protein is lost, and hence the coordinate bond is broken and the protein is eluted.

The CIM monolithic column as prepared above is equilibrated with Phosphate/MOPS [3-(N-morpholino)propanesulfonic acid]/MMA buffers [MES-MOPS-Acetate] (concentration ranging between 20mM-100mM) containing high salt (0.5M NaCl). Preferably, the column is equilibrated with 50mM phosphate buffer containing 0.5M NaCl, pH 7.0± 0.2. Constant binding and elution flow rates ranging between 3 CV/min to 18 CV/min are employed throughout the experiment. The sample (*Pichia* broth expressing factor VIII:C heavy chain concentrated by 0→80% saturation ammonium sulphate) is then injected, followed by which the column is washed with equilibration buffer (pH 6.0) until the absorbance at 280 nm reaches baseline. The said wash step is carried out to eliminate weakly bound impurities that would otherwise co-elute along with the heavy chain. Elution is carried out by lowering the pH (protonation), using 20mM-100mM of Acetate buffers containing 0.5 M NaCl (pH 4.0-6.0), preferably 50mM acetate buffers containing 0.5M NaCl at pHs 6.0, 5.0 and 4.0 respectively. This elution technique is advantageous over the alternate imidazole-based elution methods, as imidazole is toxic and would require additional desalting steps when considering the purified protein for therapeutic applications.

After the chromatographic runs, the columns are stripped off with metal ions by using ethylene diamine tetra acetate (EDTA) and the columns are regenerated with 50mM NaOH.

SDS-PAGE, followed by western blotting is performed on purified samples to confirm the presence of factor VIII:C heavy chain. About 5µg of obtained protein sample is loaded on each well of a polyacrylamide gel (10% polyacrylamide gel), and SDS-PAGE is performed. After electrophoresis, the proteins from the polyacrylamide gel are transferred to a nitrocellulose membrane using Mini Trans-Blot Cell (BIO-RAD, India). Towbin transfer buffer (25mM Tris, 200mM glycine, 0.01% SDS, 20% methanol, pH 8.3) is used to carry out the transfer from the polyacrylamide gel to the nitrocellulose membrane, at 90V for about 90 mins at a temperature of about 4°C. The membrane is washed with PBST, blocked with a blocking buffer (PBST with 5% skimmed milk powder) overnight at a temperature of about 4°C. After blocking, the membrane is incubated with primary antibody (rabbit anti-A1 or anti-C2 antiserum, along with excess of control *Pichia* broth to prevent non-specific binding) at room temperature for about one hour. The membrane is washed about 2-4 times with PBST solution after which it is incubated with secondary antibody (goat anti-rabbit IgG, HRP conjugated) at room temperature for about 1 hour. The membrane is then washed for about 2-4 times with PBST. The membrane is developed by diaminobenzidine/H₂O₂ method for detection of HRP.

The rabbit anti-A1 antiserum used for the detection of the expressed heavy chain protein by western blotting is prepared using *E. coli* expressed heavy chain terminal domain A1 domain, tagged with GST as antigen. Rabbits are immunized with the purified antigen (A1-GST). During addition of rabbit antisera to the membrane, the antiserum is incubated along with excess of *Pichia pastoris* control broth to reduce non-specific binding and background noise.

### Purification Results

Immobilized metal-ion affinity chromatography (IMAC) is employed to purify the expressed heavy chain of FVIII:C in *Pichia pastoris* clones. The rationale behind choosing IMAC is the presence of several Histidine residues on the surface of the heavy chain molecule which is confirmed based on the crystal structure of factor VIII:C.

The integrity of the purified protein is analysed over SDS-PAGE and western blotting. While sharp peaks are observed during each elution step (Fig. 4A), it is observed that the heavy chain protein is obtained to near homogeneity upon elution at pH 5.0. When analysed by SDS-PAGE and western blotting under non-reducing conditions (Fig. 4B), the heavy chain protein is sometimes observed to aggregate as a dimer, which breaks down upon reduction with DTT showing a clear band at the expected 90kDa molecular weight (Fig. 4C, lane 5).

This purified factor VIII:C heavy chain is used for reconstitution with purified light chain to further assess the coagulation activity.

### Example 4

### Activity Results of Complete Factor VIII:C Protein reconstituted from individual Light Chain and Heavy Chain

Full length B-domain deleted Factor VIII:C is regenerated from individual heavy & light chains expressed in *Pichia pastoris* expression system. Equimolar concentrations of purified heavy and light chains are reconstituted in 20mM HEPES, pH 7.0-7.4 containing 0.3M KCl, 0.01% Tween-20, 0.01% BSA, 25mM CaCl₂ and 0.5µM Cu⁺⁺ and is incubated at a temperature of about 20°C-23°C for a time-period of about 4 hours-6 hours. Following this, the coagulation activity of the reconstituted FVIII is determined by the one-stage clotting assay using FVIII-depleted plasma (Siemens Healthcare Diagnostics, USA) over IL ACL 10000 coagulation analyser (Instrumentation Laboratory, Italy). The One stage clotting assay is performed as follows-

Factor VIII-depleted plasma is dissolved in distilled or deionized water. Before use, it is allowed to stand for at least 15 minutes at a temperature of about 15 to 25°C, and then mixed carefully without foam formation. The reconstituted recombinant Factor VIII:C sample is added to FVIII deficient plasma. APTT reagents are used according to the manufacturer's instructions. 0.025M of CaCl₂ is added to the mixture. The mixture is incubated at a temperature of about 37°C and for a time-period of about 2 minutes and the reading is taken in an automated coagulation analyzer. Normal clotting time is 30-40 seconds.

The specific activity of the reconstituted recombinant FVIII:C [Light chain and Heavy chain purified using HLAC and IMAC systems respectively] is found to be 7665 IU/mg, as determined by the chromogenic assay.

### Example 5

### Purification of recombinant FVIII expressed in CHO cells using HLAC

Recombinant BDD (B-domain deleted)-FVIII [rBDD-FVIII] or functionally active FVIII expressed in CHO cell lines is purified using histidine affinity chromatography.

5 ml of harvested CHO cell culture supernatant (CSS) containing rBDD-FVIII is directly passed over a CIM-Histidine disk, pre-equilibrated with 20mM Tris, pH 6.0. After binding, the column is thoroughly washed with the same buffer, post which elution is carried out using 20mM Tris, pH 7.0 containing Glycine, Lysine, and CaCl₂.

The chromatographic profile of this purification run is depicted in Figure 5A. Two overlapping peaks are obtained (fractions A1 and A2), which upon activity analysis reveal that the factor VIII activity is found on the latter fraction (Table 4). The purification and activity results showcase a 1567 fold increase in purity of rBDD-FVIII.

**Table 4: Activity table for purification of rBDD-VIII from CHO cell lines (without intermediary wash step)**

| **Fraction** | **Amount (mg)** | **Specific Activity (IU/mg)** |
|---|---|---|
| Load | 13.32 | 0.23 |
| FT | 10.24 | 0.20 |
| Peak (A1) | 1.10 | 0.81 |
| Tail (A2) | 0.78 | 360.29 |

Further, an intermediary wash step (involving a mix of 50% binding buffer and 50% elution buffer) is introduced, in order to separate the two bands (A1 and A2) obtained above. The purification profile of the protocol having an intermediary wash step is depicted in Figure 5B. It is observed that a better separation is obtained with the introduction of intermediary wash step, resulting in a higher specific activity of the purified fraction (A3) [Table 5]. The results showcase a 6179-fold increase in purity of rBDD-FVIII.

**Table 5: Activity table for purification of rBDD-VIII from CHO cell lines (with intermediary wash step)**

| **Fraction** | **Amount (mg)** | **Specific Activity (IU/mg)** |
|---|---|---|
| Load | 13.24 | 0.23 |
| FT | 10.44 | 0.19 |
| 50% B peak (A1-A2) | 1.13 | 8.28 |
| 100% B peak (A3) | 0.13 | 1421.28 |

While preferred embodiments have been shown and described in the present invention, various modifications and substitutions may be made thereto without departing from the spirit and scope of the present invention. Accordingly, it is to be understood that apart from sources such as plasma, liver, CHO cells, *Pichia pastoris,* the presently disclosed HLAC and IMAC purification methods can be successfully employed to purify FVIII from various natural and recombinant sources including but not limiting to *Lemna gibba* and *Hansenula polymorpha.* The said purification aspects are well within the scope of the present invention.

### Example 7

### Comparative study of the efficiency of pseudobioaffinity methods of the present invention and currently known purification methods

**Table 6: Purification of FVIII from plasma/cryoprecipitate**

| Purification method | Protein Purified | Source | No. of steps | Specific Activity of product (IU/mg) | Recovery % | Purification Fold |
|---|---|---|---|---|---|---|
| CIM-His [HLAC] [Present Invention] | Full-length Factor VIII | Plasma Cryoprecipitate | One | 693 | 55% | 1386 |
| Aluminum adsorption, cold precipitation and Ion Exchange chromatography [Prior Art Method] | Full-length Factor VIII | Plasma Cryoprecipitate | Six | 175.4±37.8 | 55-65% | - |
| Anion exchange chromatography using Q-Sepharose XL [Prior Art Method] | Full-length Factor VIII | Plasma | Two | - | 40% | 54 |

**Table 7: Purification of FVIII and FVIII individual chains (heavy and light chains) from recombinant sources**

| Purification method | Protein Purified | Source | No. of steps | Specific Activity of product (IU/mg) | Purification Fold |
|---|---|---|---|---|---|
| CIM-His [HLAC] [Present Invention] | Recombinant BDD-Factor VIII | CHO cell culture supernatant | One | 1421.28 | 6179 |
| CIM-His [HLAC] [Present Invention] | FVIII Light Chain | *Pichia pastoris* | One | 7665 (upon reconstitut ion) | - |
| CIM-Cu⁺⁺ [IMAC] [Present Invention] | FVIII Heavy Chain | *Pichia pastoris* | One | 7665 (upon reconstitut ion) | - |
| Mixed mode chromatography using CaptoMMC | Recombinant Factor VIII | CHO cell culture supernatant | One (only capture; further polishing required) | 5300 | 96 |
| Affinity chromatography (VIIIselect - 13kDa recombinant peptide) [Prior Art Method] | Recombinant Factor VIII | - | Two (only capture; further polishing required) | - | - |
| Affinity chromatography (synthetic ligand L4) [Prior Art Method] | Simulated recombinant Factor VIII | Pre-purified plasma-derived FVIII in cell culture supernatant | One (only capture; further polishing required) | - | 63 |
| Mixed-mode (capto MMC), cation exchange, filtration, affinity (VIIIselect), anion exchange chromato graphy [Prior Art Method] | Recombinant Factor VIII | HEK | Eight | - | - |
| Affinity chromatography (using anti-HC & anti-LC antibodies) [Prior Art Method] | FVIII Heavy Chain and Light Chain | Baculovirus -insect cell (Sf9) system | One | 0.0285 | - |

| | | | | | |
|---|---|---|---|---|---|
| Note: The number of purification steps signifies the number of purification processes involved, and not the intermediary wash steps that are part of a particular chromatographic method. | | | | | |

The present invention thus discloses successful purification of Factor VIII protein and/or its fragments from a number of sources by employing various pseudobioaffinity based purification methods with minimal steps. Histidine Ligand Affinity Chromatography (HLAC) is used for the purification of full-length Factor VIII from plasma cryoprecipitate, for the purification of recombinant B-domain deleted Factor VIII:C expressed in various recombinant host systems and for the purification of recombinant factor VIII light chain expressed in *Pichia pastoris.* Similarly, Immobilized metal-ion affinity chromatography (IMAC) is employed for the purification of recombinant factor VIII:C heavy chain expressed in *Pichia pastoris,* for the purification of recombinant B-domain deleted Factor VIII:C expressed in various recombinant host systems etc. More importantly, the purification efficiency showcased by the methods of the present invention is far superior when compared to the presently available methods for the purification of factor VIII and hence the presently disclosed methods provide better alternatives for therapeutic applications involving factor VIII (such as treatment of hemophilia). Thus, the purification methods of the present invention successfully overcome the hurdles associated with FVIII purification in prior art and act as immense potential applications in the pharma industry to purify Factor VIII from various natural and recombinant sources.

## Claims

1. A method of purifying Factor VIII protein or fragment thereof from a sample, said method consisting of subjecting the sample to monolith based pseudo-bioaffinity purification to obtain said purified Factor VIII protein or fragment thereof, wherein the monolith based pseudo-bioaffinity purification comprises act of
a) coupling or immobilizing a monolith column with a ligand selected from a group consisting of L-histidine and chelated transition divalent metal ion selected from a group comprising copper, nickel, cobalt, zinc and iron to obtain an immobilized monolith column;
b) equilibrating the immobilized monolith column obtained in step (a) and loading the sample into the column;
c) optionally carrying out a wash step; and
d) eluting the sample to obtain the Factor VIII protein or fragment thereof.

2. The method as claimed in claim 1, wherein the Factor VIII protein or fragment thereof within the sample is naturally occurring or recombinant Factor VIII protein or fragment thereof, or a combination thereof; and wherein the natural Factor VIII protein or fragment thereof is obtained from a source selected from a group comprising plasma and liver or a combination thereof, and the recombinant Factor VIII protein or fragment thereof is obtained from a cell selected from a group comprising CHO cell, *Pichia pastoris, Lemna gibba* and *Hansunella polymorpha,* or any combination thereof.

3. The method as claimed in claim 1, wherein the Factor VIII fragment is Factor VIII Light Chain, Factor VIII heavy chain, or a combination thereof; and wherein the Factor VIII heavy chain has a molecular weight ranging from about 90 kDa to 210 kDa, and the light chain or a fragment thereof has a molecular weight of about 15 kDa to about 80 kDa.

4. The method as claimed in claim 1, wherein the method purifies Factor VIII protein or fragment thereof with purification factor value ranging from about 295 fold to about 6179 fold; and wherein the yield of purified Factor VIII protein or fragment thereof ranges from about 15 % to about 55 %.

5. The method as claimed in any of the preceding claims, wherein, the ligand is L-histidine when the naturally occurring Factor VIII protein or the recombinant Factor VIII protein or the Factor VIII light chain fragment is purified; and the ligand is chelated transition divalent metal ion selected from a group comprising copper, nickel, cobalt, zinc and iron when the recombinant Factor VIII heavy chain fragment is purified.

6. The method as claimed in claim 1 or 5, wherein the monolith based pseudo-bioaffinity purification is Histidine Ligand Affinity Chromatography (HLAC) when the ligand is L-histidine; or wherein the monolith based pseudo-bioaffinity purification is Immobilized metal-ion affinity chromatography (IMAC) when the ligand is chelated transition divalent metal ion.

7. The method as claimed in claim 1, wherein the monolith column is Convective Interaction Media (CIM) monolithic column; the coupling or immobilization is carried out in presence of coupling agent selected from a group comprising ethylene-di-amine (EDA), carbonyldiimidazole (CDI), epoxy, imino-di-acetic acid (IDA) and Tris(2-aminoethyl)amine (TREN), or any combination thereof.

8. The method as claimed in claim 1, wherein the equilibration is carried out by employing buffer selected from a group comprising cationic buffer, phosphate buffer, 3-(N-morpholino)propanesulfonic acid (MOPS) buffer and MMA buffer, or any combination thereof; and wherein the cationic buffer is selected from a group comprising Tris-HCl, and bicarbonate, or a combination thereof.

9. The method as claimed in claim 8, wherein concentration of the cationic buffer ranges from about 20 mM to about 100 mM and pH ranges from about 5.5 to about 6.5; and wherein concentration of the phosphate buffer, MOPS buffer and MMA buffer ranges from about 20 mM to about 100 mM and pH ranges from about 7.0 to about 8.0.

10. The method as claimed in claim 8, wherein the phosphate buffer or MOPS buffer or MMA buffer contain salt at concentration ranging from about 0.5 M to about 2 M.

11. The method as claimed in claim 1, wherein the elution is carried out by employing buffer selected from a group comprising cationic buffer containing calcium (II) ions, glycinate ions and lysine, acetate buffer containing sodium chloride, or a combination thereof; and wherein the cationic buffer is selected from a group comprising Tris-HCl, and bicarbonate, or a combination thereof.

12. The method as claimed in claim 11, wherein concentration of the cationic buffer ranges from about 20 mM to about 100 mM and pH ranges from about 7 to about 8; and wherein concentration of the acetate buffer ranges from about 20 mM to about 100 mM and pH ranges from about 4 to about 5.

13. The method as claimed in claim 1, wherein the wash step is carried out by employing buffer selected from a group comprising cationic buffer, phosphate buffer, 3-(N-morpholino)propanesulfonic acid (MOPS) buffer and MMA buffer, or a combination thereof; and wherein the cationic buffer is selected from a group comprising Tris-HCl, and bicarbonate, or a combination thereof.

14. The method as claimed in claim 13, wherein concentration of the cationic buffer ranges from about 10 mM to about 50 mM and pH ranges from about 7 to about 8; and wherein concentration of the phosphate buffer, 3-(N-morpholino)propanesulfonic acid (MOPS) buffer and MMA buffer ranges from about 20 mM to about 100 mM and pH ranges from about 6.0 to about 7.0.

## Patentansprüche

1. Verfahren zum Reinigen von Faktor-VIII-Protein oder Fragment davon aus einer Probe, das Verfahren darin bestehend die Probe einer monolithbasierten Pseudo-Bioaffinitäts-Reinigung zu unterziehen, um das gereinigte Faktor-VIII-Protein oder Fragment davon zu erhalten, wobei die monolithbasierte Pseudo-Bioaffinitäts-Reinigung umfasst Vorgang des
a) Koppelns oder Immobilisierens einer Monolithsäule mit einem Liganden, ausgewählt aus einer Gruppe, bestehend aus L-Histidin und chelatierten zweiwertigen Übergangsmetallion, ausgewählt aus einer Gruppe, umfassend Kupfer, Nickel, Cobalt, Zink und Eisen, um eine immobilisierte Monolithsäule zu erhalten;
b) Äquilibrierens der immobilisierten Monolithsäule, erhalten in Schritt (a) und Ladens der Probe in die Säule;
c) optionalen Durchführens eines Waschschritts; und
d) Eluierens der Probe, um das Faktor-VIII-Protein oder Fragment davon zu erhalten.

2. Verfahren, wie in Anspruch 1 beansprucht, wobei das Faktor-VIII-Protein oder Fragment davon in der Probe natürlich vorkommend oder rekombinantes Faktor-VIII-Protein oder Fragment davon oder eine Kombination davon ist; und wobei das natürliche Faktor-VIII-Protein oder Fragment davon aus einer Quelle erhalten wird, ausgewählt aus einer Gruppe, umfassend Plasma und Leber oder eine Kombination davon, und das rekombinante Faktor-VIII-Protein oder Fragment davon erhalten wird aus einer Zelle, ausgewählt aus einer Gruppe, umfassend CHO-Zelle, *Pichia pastoris, Lemna gibba* und *Hansunella polymorpha,* oder irgendeine Kombination davon.

3. Verfahren, wie in Anspruch 1 beansprucht, wobei das Faktor-VIII-Fragment Faktor-VIII leichte Kette,Faktor-VIII schwere Kette, oder eine Kombination davon ist; und wobei die Faktor-VIII schwere Kette ein Molekulargewicht reichend von etwa 90 kDa bis 210 kDa hat, und die leichte Kette oder ein Fragment davon ein Molekulargewicht von etwa 15 kDa bis etwa 80 kDa hat.

4. Verfahren, wie in Anspruch 1 beansprucht, wobei das Verfahren das Faktor-VIII-Protein oder Fragment davon mit Reinigungsfaktorwert reichend von etwa 295-fach bis etwa 6179-fach reinigt; und wobei die Ausbeute an gereinigtem Faktor-VIII-Protein oder Fragment davon von etwa 15 % bis etwa 55 % reicht.

5. Verfahren, wie in einem der vorhergehenden Ansprüche beansprucht, wobei der Ligand L-Histidin ist, wenn das natürlich vorkommende Faktor-VIII-Protein oder das rekombinante Faktor-VIII-Protein oder das Faktor-VIII leichte Ketten Fragment gereinigt wird; und der Ligand chelatisiertes zweiwertiges Übergangsmetallion ist, ausgewählt aus einer Gruppe, umfassend Kupfer, Nickel, Cobalt, Zink und Eisen, wenn das rekombinante Faktor-VIII schwere Ketten Fragment gereinigt wird.

6. Verfahren, wie in Anspruch 1 oder 5 beansprucht, wobei die monolithbasierte Pseudo-Bioaffinitäts-Reinigung Histidin-Ligand-Affinitätschromatographie (HLAC) ist, wenn der Ligand L-Histidin ist; oder wobei die monolithbasierte Pseudo-Bioaffinitäts-Reinigung immobilisierte Metallionen-Affinitätschromatographie (IMAC) ist, wenn der Ligand chelatiertes zweiwertiges Übergangsmetall ist.

7. Verfahren, wie in Anspruch 1 beansprucht, wobei die Monolithsäule Convective Interaction Media (CIM) Monolithsäule ist; die Kupplung oder Immobilisierung in Gegenwart eines Kupplungsmittels durchgeführt wird, ausgewählt aus einer Gruppe , umfassend Ethylen-Diamin (EDA), Carbonyldiimidazol (CDI), Epoxid, Iminodiessigsäure (IDA) und Tris(2-Aminoethyl)amin (TREN), oder irgendeine Kombination davon.

8. Verfahren, wie in Anspruch 1 beansprucht, wobei die Äquilibrierung durchgeführt wird, indem Puffer verwendet wird, ausgewählt aus einer Gruppe, umfassend kationischen Puffer, Phosphatpuffer, 3-(N-Morpholino)propansulfonsäure (MOPS)-Puffer und MMA-Puffer, oder irgendeine Kombination davon; und wobei der kationische Puffer aus einer Gruppe ausgewählt ist, umfassend Tris-HCl, und Bicarbonat, oder eine Kombination davon.

9. Verfahren, wie in Anspruch 8 beansprucht, wobei Konzentration des kationischen Puffers von etwa 20 mM bis etwa 100 mM reicht und pH von etwa 5,5 bis etwa 6,5 reicht; und wobei Konzentration des Phosphatpuffers, MOPS-Puffers und MMA-Puffers von etwa 20 mM bis etwa 100 mM reicht und pH von etwa 7,0 bis etwa 8,0 reicht.

10. Verfahren, wie in Anspruch 8 beansprucht, wobei der Phosphatpuffer oder MOPS-Puffer oder MMA-Puffer Salz in Konzentration reichend von etwa 0,5 M bis etwa 2 M enthalten.

11. Verfahren, wie in Anspruch 1 beansprucht, wobei die Elution durchgeführt wird, indem Puffer verwendet wird, ausgewählt aus einer Gruppe, umfassend kationischen Puffer, enthaltend Calcium-(II)-Ionen, Glycinationen und Lysin, Acetatpuffer, enthaltend Natriumchlorid, oder eine Kombination davon; und wobei der kationische Puffer aus einer Gruppe ausgewählt ist, umfassend Tris-HCl, und Bicarbonat, oder eine Kombination davon.

12. Verfahren, wie in Anspruch 11 beansprucht, wobei Konzentration des kationischen Puffers von etwa 20 mM bis etwa 100 mM reicht und pH von etwa 7 bis etwa 8 reicht; und wobei Konzentration des Acetatpuffers von etwa 20 mM bis etwa 100 mM reicht und pH von etwa 4 bis etwa 5 reicht.

13. Verfahren, wie in Anspruch 1 beansprucht, wobei der Waschschritt durchgeführt wird, indem Puffer verwendet wird, ausgewählt aus einer Gruppe, umfassend kationischen Puffer, Phosphatpuffer, 3-(N-Morpholino)propansulfonsäure (MOPS)-Puffer und MMA-Puffer, oder eine Kombination davon; und wobei der kationische Puffer aus einer Gruppe ausgewählt ist, umfassend Tris-HCl, und Bicarbonat, oder eine Kombination davon.

14. Verfahren, wie in Anspruch 13 beansprucht, wobei Konzentration des kationischen Puffers von etwa 10 mM bis etwa 50 mM reicht und pH von etwa 7 bis etwa 8 reicht; und wobei Konzentration des Phosphatpuffers, 3-(N-Morpholino)propansulfonsäure (MOPS)-Puffers und MMA-Puffers von etwa 20 mM bis etwa 100 mM reicht und pH von etwa 6,0 bis etwa 7,0 reicht.

## Revendications

1. Procédé pour purifier une protéine de facteur VIII ou un fragment de celle-ci à partir d'un échantillon, ledit procédé consistant à soumettre l'échantillon à une purification par pseudo-bioaffinité à base de monolithe pour obtenir ladite protéine de facteur VIII purifiée ou un fragment de celle-ci, dans lequel la purification par pseudo-bioaffinité à base de monolithe comprend les opérations suivantes :
a) couplage ou immobilisation d'une colonne monolithique avec un ligand choisi dans l'ensemble constitué par la L-histidine et un ion de métal divalent de transition chélaté choisi dans l'ensemble comprenant le cuivre, le nickel, le cobalt, le zinc et le fer pour que soit obtenue une colonne monolithique immobilisée ;
b) équilibrage de la colonne monolithique immobilisée obtenue dans l'étape (a) et chargement de l'échantillon dans la colonne ;
c) éventuellement mise en oeuvre d'une étape de lavage ; et
d) élution de l'échantillon pour que soit obtenu la protéine de facteur VIII ou un fragment de celle-ci.

2. Procédé selon la revendication 1, dans lequel la protéine de facteur VIII ou le fragment de celle-ci dans l'échantillon est une protéine de facteur VIII naturelle ou recombinante ou un fragment de celle-ci, ou une combinaison de ceux-ci ; et dans lequel la protéine de facteur VIII naturelle ou le fragment de celle-ci est obtenu à partir d'une source choisie dans l'ensemble comprenant le plasma et le foie ou une combinaison de ceux-ci, et la protéine de facteur VIII recombinante ou le fragment de celle-ci est obtenu à partir d'une cellule choisie dans l'ensemble comprenant une cellule CHO, *Pichia pastoris, Lemna gibba* et *Hansunella polymorpha,* ou l'une quelconque de leurs combinaisons.

3. Procédé selon la revendication 1, dans lequel le fragment de facteur VIII est une chaîne légère de facteur VIII, une chaîne lourde de facteur VIII, ou une combinaison de celles-ci ; et dans lequel la chaîne lourde de facteur VIII a un poids moléculaire situé dans la plage allant d'environ 90 kDa à 210 kDa, et la chaîne légère ou un fragment de celle-ci a un poids moléculaire d'environ 15 kDa à environ 80 kDa.

4. Procédé selon la revendication 1, dans lequel le procédé purifie la protéine de facteur VIII ou un fragment de celle-ci avec une valeur de facteur de purification située dans la plage allant d'environ 295 fois à environ 6179 fois ; et dans lequel le rendement en protéine de facteur VIII ou fragment de celle-ci est situé dans la plage allant d'environ 15 % à environ 55 %.

5. Procédé selon l'une quelconque des revendications précédentes, dans lequel le ligand est la L-histidine quand la protéine de facteur VIII naturelle ou la protéine de facteur VIII recombinante ou le fragment de chaîne légère de facteur VIII est purifié ; et le ligand est un ion de métal divalent de transition chélaté choisi dans l'ensemble comprenant le cuivre, le nickel, le cobalt, le zinc et le fer quand le fragment de chaîne lourde de facteur VIII recombinant est purifié.

6. Procédé selon la revendication 1 ou 5, dans lequel la purification par pseudo-bioaffinité à base de monolithe est une chromatographie d'affinité sur ligand histidine (HLAC) quand le ligand est la L-histidine ; ou dans lequel la purification par pseudo-bioaffinité à base de monolithe est une chromatographie d'affinité sur ions métalliques immobilisés (IMAC) quand le ligand est un ion de métal divalent de transition chélaté.

7. Procédé selon la revendication 1, dans lequel la colonne monolithique est une colonne monolithique à milieu d'interaction convectif (CIM) ; le couplage ou l'immobilisation est effectué en présence d'un agent de couplage choisi dans l'ensemble comprenant l'éthylènediamine (EDA), le carbonyldiimidazole (CDI), l'époxy, l'acide iminodiacétique (IDA) et la tris(2-aminoéthyl)amine (TREN), ou l'une quelconque de leurs combinaisons.

8. Procédé selon la revendication 1, dans lequel l'équilibrage est effectué par l'emploi d'un tampon choisi dans l'ensemble comprenant un tampon cationique, un tampon phosphate, un tampon acide 3-(N-morpholino)propanesulfonique (MOPS) et un tampon MMA, ou l'une quelconque de leurs combinaisons ; et dans lequel le tampon cationique est choisi dans l'ensemble comprenant le Tris-HCl et le bicarbonate, ou l'une quelconque de leurs combinaisons.

9. Procédé selon la revendication 8, dans lequel la concentration du tampon cationique est située dans la plage allant d'environ 20 mM à environ 100 mM et le pH est situé dans la plage allant d'environ 5,5 à environ 6,5 ; et dans lequel la concentration du tampon phosphate, du tampon MOPS et du tampon MMA est située dans la plage allant d'environ 20 mM à environ 100 mM et le pH est situé dans la plage allant d'environ 7,0 à environ 8,0.

10. Procédé selon la revendication 8, dans lequel le tampon phosphate ou le tampon MOPS ou le tampon MMA contient un sel à une concentration située dans la plage allant d'environ 0,5 M à environ 2 M.

11. Procédé selon la revendication 1, dans lequel l'élution est effectuée par l'emploi d'un tampon choisi dans l'ensemble comprenant un tampon cationique contenant des ions calcium (II), des ions glycinate et de la lysine, un tampon acétate contenant du chlorure de sodium, ou l'une de leurs combinaisons ; et dans lequel le tampon cationique est choisi dans le groupe comprenant le Tris-HCl et le bicarbonate, ou l'une de leurs combinaisons.

12. Procédé selon la revendication 11, dans lequel la concentration du tampon cationique est située dans la plage allant d'environ 20 mM à environ 100 mM et le pH est situé dans la plage allant d'environ 7 à environ 8 ; et dans lequel la concentration du tampon acétate est situé dans la plage allant d'environ 20 mM à environ 100 mM et le pH est situé dans la plage allant d'environ 4 à environ 5.

13. Procédé selon la revendication 1, dans lequel l'étape de lavage est effectuée par l'emploi d'un tampon choisi dans l'ensemble comprenant un tampon cationique, un tampon phosphate, un tampon acide 3-(N-morpholino)propanesulfonique (MOPS) et un tampon MMA, ou l'une de leurs combinaisons ; et dans lequel le tampon cationique est choisi dans l'ensemble comprenant le Tris-HCl et le bicarbonate, ou l'une de leurs combinaisons.

14. Procédé selon la revendication 13, dans lequel la concentration du tampon cationique est située dans la plage allant d'environ 10 mM à environ 50 mM et le pH est situé dans la plage allant d'environ 7 à environ 8 ; et dans lequel la concentration du tampon phosphate, du tampon acide 3-(N-morpholino)propanesulfonique (MOPS) et du tampon MMA est située dans la plage allant d'environ 20 mM à environ 100 mM et le pH est situé dans la plage allant d'environ 6,0 à environ 7,0.
